(19)

Europäisches
Patentamt
European
Patent Office
Office européen
des brevets

(11) **EP 3 020 406 A1**

(12) **EUROPEAN PATENT APPLICATION**

(43) Date of publication:
**18.05.2016  Bulletin 2016/20**

(21) Application number: **15194312.3**

(22) Date of filing: **12.11.2015**

(51) Int Cl.:
**A61K 36/258** (2006.01)     **A61P 1/16** (2006.01)
**A61K 31/575** (2006.01)     **A23L 33/105** (2016.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **12.11.2014   KR 20140156917**

(71) Applicant: **Cj Cheiljedang Corporation
Seoul 04560 (KR)**

(72) Inventors:
• **SHIN, Heung Sop
Gyeonggi-do 13835 (KR)**
• **SHIN, Hwa Soo
Gyeonggi-do 13835 (KR)**

(74) Representative: **Goddard, Christopher Robert
Dehns
St Bride's House
10 Salisbury Square
London
EC4Y 8JD (GB)**

(54) **PHARMACEUTICAL COMPOSITION AND HEALTH FUNCTIONAL FOOD CONTAINING RED GINSENG CONCENTRATE HAVING ENHANCED COMPOUND K FOR PREVENTING AND TREATING NON-ALCOHOLIC FATTY LIVER DISEASE**

(57)     The present invention relates to a pharmaceutical composition and a health functional food composition for preventing and treating non-alcoholic fatty liver disease, which comprises red ginseng concentrate having enhanced Compound K as an effective ingredient by using an enzyme conversion technique.

EP 3 020 406 A1

**Description**

[Technical Field]

The present invention relates to a pharmaceutical composition containing a red ginseng concentrate having enhanced compound K as an effective ingredient for preventing and treating non-alcoholic fatty liver disease and a health functional food containing the red contrate having enhanced compound K as the effective ingredient, for preventing and treating non-alcoholic fatty liver disease.

[Background Art]

Ginseng (Panax ginseng C.A. Mayer) is a plant belonging to genus of *Araliaceau ginseng,* and has been used for a drug in China from B.C., and has been used for a trade and drug from the period of the Three States, and is being widely used as a medicinal herbs or health functional food in various fields until now.

Ginseng comprises about 3-5 percent of prosaponin called as Ginsenoside, the Ginsenoside is a representative physiological active substance of ginseng and about 33 kinds of it are reported. Since ginsenoside of ginseng is connected to a constitutive component of a polymer, it cannot be easily absorbed into a body after ingestion and thus is digested by a microorganism inhabiting in an intestine and then is absorbed into the body. That is, a ginsenoside form in which the glucose bonded to ginsenoside is digested is finally absorbed into the body, each of ginsenosides exhibits a physiological activity being different from each other.

Meanwhile, 20-O-$\beta$-glucopyranosyl-20(S)-protopanaxadiol (hereinafter, referred to as Compound K) is one of protopanaxadiol (PPD)-based saponin in which a glucose is bonded to carbon at the position of 20 of aglycon and is a convertant produced from ginsenoside Rb1, Rc, Rb2 by an intestinal bacteria (Hasegawa, H. Et al., Planta Medica, 62 :453-457(1996)). Compound K is known as having an inhibition for the proliferation and metastasis of cancer cells, anti-aging, anti-allergy and immunostrengthen function, but is not included in ginseng or red ginseng itself, and can be obtained as a matabolic product in a body by the intestinal bacteria. But, it is reported that since the kinds of the intestinal bacteria and metabolic ability are different from person to person, there is a difference in an absorption and efficacy depending on the individual (Hasegawa, H. et al., Planta Medica, 63(5) :436-440(1997).

As a method for converting ginseng saponin into compound K, a heat treatment (Kitagawa et al., Yakugaku Zasshi., 103 :612-622(1983) ; Kwon et al., J.Chromatography A., 921 :335-339(2001) ; Park, Food Ind. Nutr., 9 :23-27(2004)), an acid treatment (Han et al., Planta medica., 44: 146-149(1982), Bae et al., Arch Pharm Res. 27(1) :61-67(2004)), an alkali treatment (Chen et al., Chem. Pharm. Bull. 35 :1653-1655 (1987) ; Im et al., Kor J Ginseng Sci. 19 :291-294 (1995)), an organic synthesis (Anufriev et al, Carbohydr Res. 304 :179-182 (1997)), a conversion method by an enzyme of a microorganism, etc., are known in the art. The enzyme conversion method by using the enzyme among these methods can be considered as the most efficient gingeng saponin conversion method, due to that it can selectively convert a substrate by specfically functioning to the substrate while making almost no reaction byproduct, it can proceed the reaction under the reletively safe condition, it exhibits a high efficiency, etc.

In addition, as presented in the Korean laid-open Patent Publication No. 10-2013-0085970 as a previous study of the present invention, the optimal preparation method of ginseng concentrate having enhanced Compound K in which compound K is enhanced, by using the enzyme conversion method has been reported.

Liver is responsible for a detoxification function and also for a synthesis, metabolism, storage and redistribution metabolic procedures of carbohydrate, protein, lipid, etc., being a majour nutrient, and have a function keeping a metabolism homeostatis of a body through such procedures. But, the liver function can be damaged by various reasons such as a virus, inflammation, heavy drinking of alcohol, drug, overwork, etc.

As a symptom considered as the major reason of a damage of liver function and a liver diorder, a fatty liver can be mentioned, the frequency of which is increasing by an influence of recent westernized dietary life habitat, drinking and stress, etc. The fatty liver can be said as a symptom that a neutral fat is excessively accumulated in the liver (5% or more of the weight of liver) by the influence of drinking or excessive intake of fat and stress, etc. The fatty liver can be kept in a state which does not develop into any disorder and does not make a great influence to a health, but since it can be developed into diseases such as liver inflammation and liver cirrhosis, etc., it needs a sustained care and management.

Aftty liver disease is divided into alcoholic fatty liver disease due to the excessive alcohol intake, and non-alchoholic fatty Iver disease (NAFLD) not due to that.

Non-alcoholic fatty disease is a disorder in which neutral fat is accumulated in the liver regardless of drinking, and means a series of disorder group including simple steatosis with only the excessive accumulation of fat in the liver, non-alcoholic steatohepattis (NASH) accompanying necrosis, inflammation and fibrosis of liver cell, and liver cirrhosis (LC) which is more progressing form thereof.

A mobidity of non-alcoholic fatty liver disease is recently increasing in not many western countries, but also in Korea, is related to an increse of adult and child obesity, and is reported about 20~30% of whole population in advanced country, although there is a difference from country to country.

Recently, non-alcoholic fatty liver disease is considered as being one type of metabolic syndromes such as obesity, hypertension, type II diabetes mellitus, lipid metabolism disorder based on insulin resistance. Non-alcoholic fatty liver disease can be a simple lipidosis, but there is a problem that non-alcoholic fatty liver inflammation is occoured in about 10~20% in patients with non-alcoholic fatty liver disease, and about 9 ~ 25% of amont the patients is developed into cirrhosis.

Non-alcoholic fatty liver disease can be usually recovered by properly intaking nutrients and regulating the relevant metabolic disorders, but a prognosis becomes poor under neglecting. Therefore, in order to prevent the develoement of non-alcoholic fatty liver disease into cirrhosis, it should be prevented that the fat is accumulated in the liver, but it is under a circumstance that there is no way except for the method for reducing intaking calories or increasing calories consumption.

[Disclosure]

[Technical Problem]

Under said circumstances, as a result that the inventors of the present invention tried to provide a composition which can prevent and treat non-alcoholic fatty liver disease, red ginseng concentrate having enhanced compound K from ginseng was prepared and the composition for preventing and treating non-alcoholic fatty liver disease, which comprises it, is identified as having effects for inhibiting an accumulation of fat in the liver of fatty liver disease caused through a high-fat diet in an animal test, and thus, the present invention has been completed.

The other object of the present invention is to provide a pharmaceutical composition for preventing and treating non-alcoholic fatty liver disease, which comprises red ginseng concentrate having enhanced compound K as an effective ingredient.

The other objection of the present invention is also to provides a health functional food containing red ginseng concentrate having enhanced compound K for preventing and treating non-alcoholic fatty liver disease.

[Technical Solution]

In order to achieve the above objects, the present invention provides a pharmaceutical composition for preventing and treating non-alcoholic fatty liver disease, which comprises red ginseng concentrate having enhanced compound K as an effective ingredient.

The present invention provides a health functional food containing red ginseng concentrate having enhanced compound K for preventing and treating non-alcoholic fatty liver disease.

[Advantageous Effects]

The present invention has effects providing a pharmaceutical composition and health functional food for preventing and treating non-alcoholic fatty liver disease, which comprises red ginseng concentrate having enhanced compound K safety of which is increased by extracting it only with water and ethyl acohol, and said composition can provide an effect inhibiting non-alcoholic fatty liver disease through the reduction of contens of neutral lipid (triglycerides, TG) in blood and the liver tissue.

[Description of Drawings]

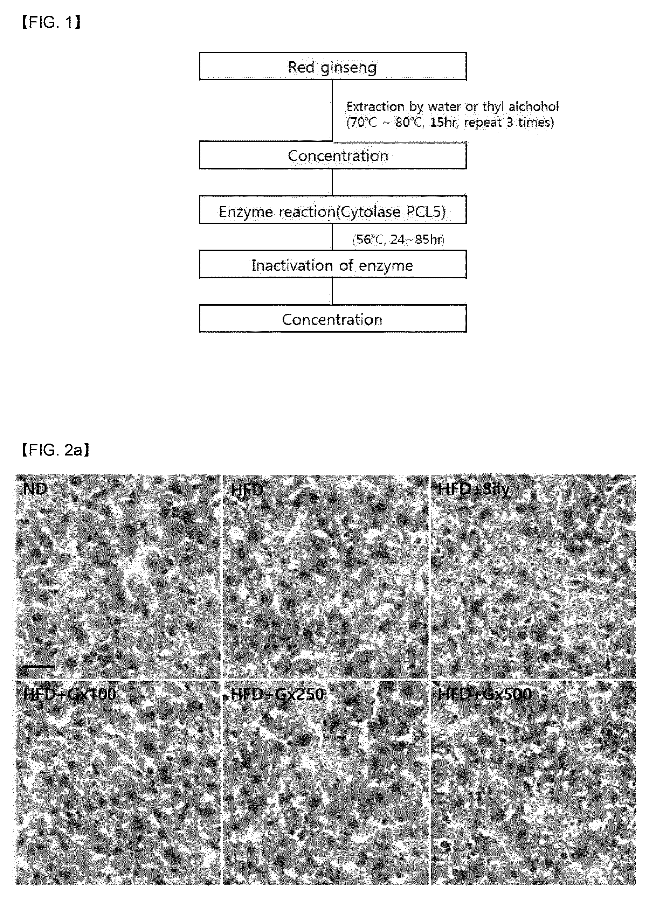

FIG.1 represents a procedure for preparing red ginseng concentrate having enhanced compound K by an enzyme conversion technique.

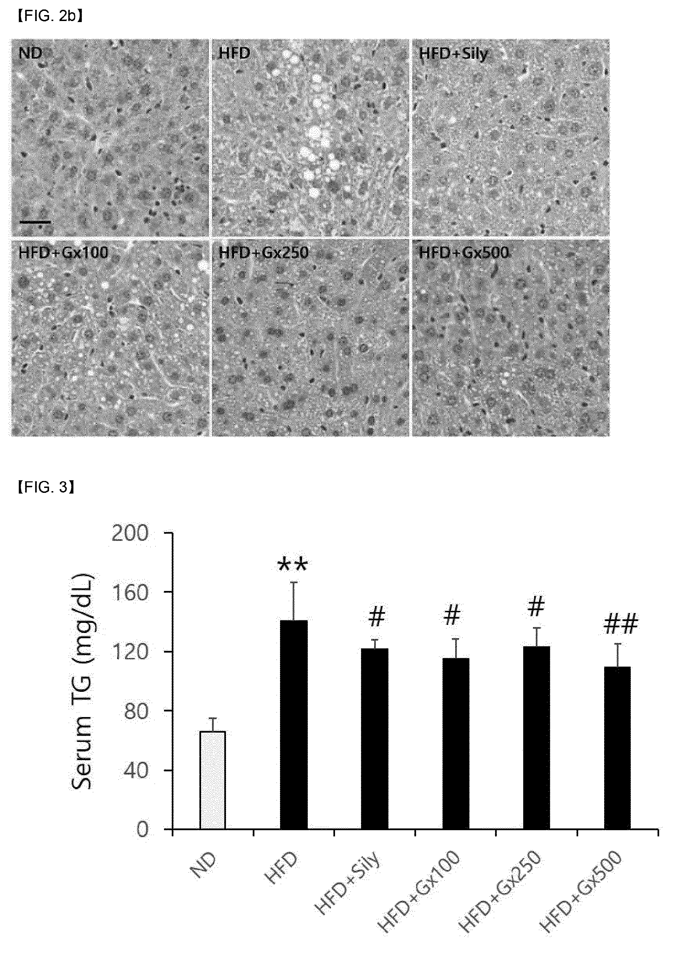

FIG.2a and 2b represent a histopathological change of experimental animals for Normal Diet (ND) control, High-Fat Diet (HFD) control, High-Fat Diet + silymarin treatment group (HFD + sily) and a treatment group of High-Fat Diet + Ginseng concentrate having enhanced Compound K at each concentrations according to the present invention (HFD + Gx100, Gx250, Gx500). FIG. 2a represents results of photography after staining Oil Red O, respectively. FIG. 2b represents results of photography after hematoxylin-eosin stain, respectively.

FIG.3 represents a change of a neutral lipid among biochemical index of experimental animals according to an administration of Normal Diet (ND) control, High-Fat Diet (HFD) control, High-Fat Diet (HFD) + silymarin and, administration of High-Fat Diet (HFD) + Ginseng concentrate having enhanced Compound K at each concentrations according to the present invention in the fatty liver animal models in vivo.
Normal Diet (ND) vs. High-Fat Diet (HFD): *$p < 0.05$, **$p < 0.01$.

High-Fat Diet (HFD) vs. High-Fat Diet + samples (HFD + samples): #$p < 0.05$, ##$p < 0.01$.

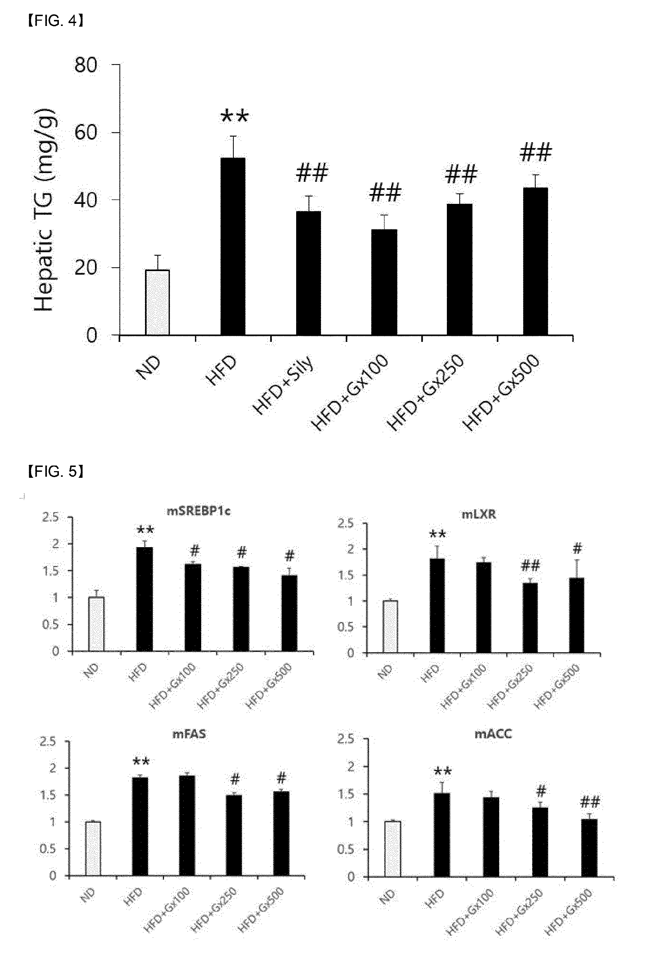

FIG.4 represents a change for contents of a neutral lipid in the liver tissue of experimental animals according to an administration of Normal Diet (ND) control, High-Fat Diet (HFD) control, High-Fat Diet (HFD) + silymarin, and administration of High-Fat Diet (HFD) + Ginseng concentrate having enhanced Compound K at each concentrations according to the present invention in the fatty liver animal models in vivo.
ND vs. HFD: *$p < 0.05$, **$p < 0.01$.
HFD vs. HFD + samples: #$p < 0.05$, ##$p < 0.01$.
FIG.5 represents a result for the gene expression assay for proteins (SREBP-1c, LXR, FAS and ACC) related to the lipid metabolism in liver tissue.
ND vs. HFD: *$p < 0.05$, **$p < 0.01$.
HFD vs. HFD + samples: #$p < 0.05$, ##$p < 0.01$.

[Best Mode for Invention]

Hereinafter, the present invention will be illustrated in detail.

As the first embodiment of the present invention, the present invention provides a pharmaceutical composition for preventing and treating non-alcoholic fatty acid liver disease, which comprises red ginseng concentrate having enhanced Compound K component as an effective ingredient.

The red ginseng concentrate having enhanced Compound K component as used in the present invention can be prepared according to the method comprising a step for obtaining red ginseng concentrate by using water or ethyl alcohol as the extractive solvent; and a step for obtaining red ginseng concentrate having enhanced Compound K component by using the enzyme conversion technique. For example, it can be prepared according to the method as described in Korean laid-open Patent Publication No. 10-2013-0085970.

The present invention uses water or ethyl alcohol which is the solvent allowed under National Food Sanitation Law as an extractive solvent. Therefore, red ginseng concentrate having enhanced Compound K according to the present invention can be safely used in making it into Food•Medicine, unlike the conventional method of the prior art using an organic solvent as the extractive solvent.

The above alcohol is preferably 70 ~ 90% ethyl alcohol, more preferably is used as 80% ethyl alcohol.

Ginseng saponin in red ginseng extract extracted to convert red ginseng extract into Compound K is reacted with polysaccharide or ethyl alcohol breakdown enzyme in the present invention. The above polysaccharide or ethyl alcohol breakdown enzyme can be cellulase, pectinex, or polysaccharide or alcohol breakdown enzyme having β-glucosidase titer, and is preferably cytolase PCL5 having a higher conversion yield of ginsenoside Compound K and is derived from *Aspergillus niger.*

It is preferable that the above breakdown enzyme is used as an amount of 2% to the amount of the enzyme breakdown substrate.

In the present invention, it is preferable that the contents of Compound K is enhanced to the level of 0.5 ~ 1.5mg/g.

Red ginseng concentrate having enhanced the above Compound K can be comprised as 0.1 to 99 % by weight to the total of weight of the pharmaceutical composition.

The above pharmaceutical composition of the present invention can be formulated as various forms including a pharmaceutically acceptable carrier, for example, a formulation for oral such as powder, granule, tablet, capsule, suspension, emulsion, syrup, aerosol, etc., forms of an external preparation, suppository and sterilized injection solution. In particular, it can be preferably prepared into the formulation for oral administration.

The above pharmaceutically acceptable carrier includes lactose, dextrose, sucrose, sorbitol, manitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, prophylhydroxybenzoate, talc, magnesium stearate and mineral oil, etc.

In addition, the above pharmaceutical composition according to the present invention comprises diluents or excipients such as filler, extender, binder, wetting agent, disintegrating agent, surfactant, etc.

The solid preparation for oral administration comprises tablet, pill, powder, granule, capsule, etc., and such solid preparation can comprise at least one or more of excipients, for example, starch, calcium carbonate, sucrose or lactose, gelatin, etc. and can comprise lubricants such as magnesium stearate, talc, etc.

The liquid preparation for oral comprises suspension, solution, emulsion, syrup, etc. and can comprise diluents such as water, liquid parafin, etc., wetting agent, sweetening agent, flavoring agent, preservatives, etc.

A parenteral preparation comprises an aqueous sterilized solution, non-aqueous solution, suspension, emulsion, freeze drying agent and suppository agent, and comprises non-aqueous solution, propylene glycol, polyethylene glycol, a vegetable oil such as olive oil, and an injectable ester such as ethyloleate, as a suspending agent.

As a base of a suppository agent, witepsol, macrogol, tween 61, cacao butter, laurin oil, glycero gelatin, etc. can be used.

When experimental animals are bred only with high fatty diet for 11 weeks in the present invention, the accumulation of fat in the liver cell is greatly increased in said animals. But, when experimental animals are administered with the pharmaceutical composition comprising red ginseng concentrate having enhanced Compound K according to the present invention as an effective component during breeding them with only high fatty diet, the accumulation of fat in hepatocytes of such animals is greatly reduced than those bred with only high fatty diet. Therefore, it can be identified that the pharmaceutical composition comprising red ginseng concentrate having enhanced Compound K according to the present invention inhibits the occurrence of non-alcoholic fatty liver disease caused by high fatty diet in the experimental animals.

As the second embodiment of the present invention, the present invention provides the health functional food for preventing and treating non-alcoholic fatty liver disease, which comprises the red ginseng concentrate having enhanced compound K as the effective ingredient.

The red ginseng concentrate having enhanced compound K can be preferably comprised as 0.1 to 99 % by weight to the total weight of the health functional food composition.

The health functional food composition of the present invention can be used as the health functional food. The term "the health functional food" means the food which is prepared and processed with raw materials or components having functionality useful for a human body according to Law No6727 regarding Health Functional Food, the term, "functional" means the intake intended for obtaining effects useful for regulating nutrients to the structure and function of the human body or for obtaining effects useful for health uses such as physiological functions, etc.

The health functional food composition of the present invention can comprise conventional food additives, and is determined by a standard or criterion regarding the relevant article according to general provisions of Korean Food Additives Codex and General Test Method approved by the Korean Food & Drug Administration.

Items listed on the "Korean Food Additives Codex" can be mentioned, for example, a chemical composite such as ketone, glycine, potassium citrate, nicotinic acid, cinnamic acid, etc., natural additives such as persimmon Color, Glycyrrhiza extract, crystalline cellulose, Kaoliang color, Guar gum, etc., and mixed preparations such as L-glutamic acid sodium agent, alkali agents for noodles, preservative formulation, tar color formulation, etc.

The health functional food composition of the present invention can be prepared in any of the forms selected from the group consisting of beverage, capsule, bolus and granule.

In the case of health functional food in the form of beverage, if necessary, it can comprises preservatives, stabilizers, emulsions, dispersions, flavoring agent, coloring agent, etc.

In the case of health functional food in the form of capsule, a hard capsule can be prepared by filling a mixture of additives such as herbal medicine extracts and excipients, etc., or granules thereof or coated granules into the conventional hard capsule, and a soft capsule can be prepared by filling the mixture of additives such as herbal medicine extracts and excipients, etc. into the capsule base such as gelatin, etc. The above soft capsule can comprise plasticizer such as glycerine or sorbitol, etc., coloring agent, preservatives, etc., if necessary.

The health functional food in the form of bolus can be prepared by shaping the mixture of excipients, binding agent, integrating agent, etc. into the herbal medicinal extract by the proper method, and can be covered by coating it with white sugar or other proper coating agents, or with dusting powder, such as starch, talc or proper materials.

The health functional food in the form of granule can be prepared in the form of granular with the mixture of excipients, binding agents, integrating agents, etc. into the herbal medicinal extract by the proper method, and can comprise flavoring agents, corrigents, etc., if necessary. A definition for above terms excipients, binding agents, disintegrating agents, glydents, corrigents, flavoring agents, etc. is described in literatures known in the relevant art and includes those having the same or similar ones in the functions, etc. (Explanation of the Korean Pharmacopoeia, Munsung Company, Korean College of Pharmacy Conference, revised Edition Volume. 5, p33-p48, 1989).

Hereinafter, the present invention will be explained more detail by the Working examples and Experimental examples as below. But, these Working examples and Experimental examples are intended to help the understanding of the present invention, and the scope of the present invention is not limited to them by any sense.

**[Examples]**

**Example 1: The preparation of red ginseng concentrate having enhanced Compound K**

The ginseng used in the present study is a dried red ginseng (4 year old root), and the red ginseng extract was prepared by adding water or ethyl alcohol being 10 times of raw materials (w/v) and reflux-extracting it three times at 70~80°C, 200 rpm, for 15 hours, and then concentrate having 70 brixs or more was prepared by double cooling the red ginseng extract in water bath at a room temperature or a low temperature, filtering and concentrating it under the reduced pressure.

The concentrate was diluted with 10 times (w/v) of water, was regulated to pH 4.3 by adding citric acid, and then, added 2% (v/v) of an enzyme cytolase PCL5 (DSM Company, France) and was reacted it at 56°C, 200 rpm for 24 ~ 85 hours.

Next, the enzyme was inactivated by heating the concentrate in boiling water bath for 30 minutes not to occur the reaction any more, and then red ginseng concentrate having enhanced Compound K was prepared by concentrating it in the reduced pressure. The procedure mentioned above is represented in FIG. 1.

**Experimental Example 1: Verification of the efficacy through fatty liver animal models *in vivo***

In order to study the function of red ginseng concentrate having enhanced Compound K for improvement in non-alcoholic fatty liver, 5 weeks-old C57BL/6 male mouse was used to construct high-fat diet induced obesity animal model and to proceed *in vivo* experiment.

This model is mainly used in a rodent and it is a general situation that effects are verified by inducing obesity with high-fat diet in the model and then administering a sample to be tested to the model. Therefore, we tried to review the efficacy through the animal experiment for red ginseng concentrate having enhanced Compound K prepared by the above Example 1.

As indicated in Table 1 below, experimental animals were divided into a total of six (6) groups and were administered with the defined diets and samples for 11 weeks. Normal diet (ND) or High Fat Diet (HFD) as the control, High Fat Diet + sylimarin (HFD + Sily)(200mg/kg/day) as a positive control, and High Fat Diet + red ginseng concentrate having enhanced Compound K (HFD + Gx) as an experimental group, with separating low, medium and high concentrations (100, 250, 500mg/kg/day), respectively, were administered to the animals orally.

Wherein, silymarin has the most excellent efficacy among all kinds of components which treat a liver and in particular, is known as having the efficacy for a fatty liver, hepatitis, cirrhosis, etc.

[Table 1]

| Experiment Group | Diet + Sample treatment condition |
|---|---|
| ND | Normal diet control |
| HFD | High Fat Diet control |
| HFD + Sily | High Fat Diet control + silymarin 200mg/kg day |
| HFD + Gx100 | High Fat Diet + red ginseng concentrate 100mg/kg/day |
| HFD + Gx250 | High Fat Diet + red ginseng concentrate 250mg/kg/day |
| HFD + Gx500 | High Fat Diet + red ginseng concentrate 500mg/kg/day |

**1-1 Changes of a weight of body and liver in the experimental animals**

The weight of body for the animals was determined at 1 time interval per every week for 11 weeks, and Student T-test for statistic analysis was performed. High Fat Diet control (HFD) was Normal diet control (ND), but High Fat Diet + silymarin treatment group (HFD+Sily) or High Fat Diet + red ginseng concentrate having enhanced Compound K was compared with High Fat Diet (HFD) control. A trend for the change of average weight of body in each of the experimental groups is exhibited in Table 2 below.

[Table 2]

|  | ND | HFD | HFD+Sily | HFD+Gx100 | HFD+Gx250 | HFD+Gx500 |
|---|---|---|---|---|---|---|
| The initial weight (g) | 20.0± 0.5 | 20.0± 0.3 | 20.0± 0.5 | 19.9± 0.3 | 19.9± 0.3 | 20.3± 0.6 |
| The final weight (g) | 24.0± 1.0 | 35.3± 1.9** | 30.8± 2.6# | 32.9± 2.7 | 32.5± 5.1 | 33.3± 2.7 |
| The increasing amounts of the weight (g) | 3.9± 1.0 | 14.9± 1.7** | 10.8± 2.7# | 13.1± 2.7 | 12.7± 5.1 | 13.0± 2.4 |
| Normal diet vs. High Fatty diet: *($p<0.05$), **($p<0.01$)<br>High Fatty diet vs. High Fatty diet + sample: #($p<0.05$), ##($p<0.01$) | | | | | | |

The average body weights of the experimental animals had no significant differences at the time of beginning of the Experiment, but the body weight of High Fatty diet (HFD) control exhibited the stiff increasing over the Normal diet (ND) control during a period of experiment. Such result means that an energy efficiency of High Fatty diet is very high, in the case of High Fatty diet (HFD) control, and it means the possibility that an overweight may form the fatty liver is also high.

As indicated in Table 2 above, in the case of the final average weight of body, it was exhibited that High Fatty diet control (HFD) was 35.3±1.9g and was about 47% high over the Normal diet control of 24.0±1.0g. In the case of red ginseng concentrate having enhanced Compound K experiment group (HFD+Gx), when comparing it with High Fatty diet control (HFD), there was no significant difference in that the final average weight of body of HFD+Gx100(32.9±2.7g) was about 6.8%, HFD+Gx250 (32.5±5.1g) was about 7.9%, HFD+Gx500(33.3±2.7g) was about 5.7%, but the tendency can be identified that the average weight of body was decreased.

After completing the experiment for 11 weeks, animal were sacrificed and the liver was removed from all of the animals, its weight was determined and the statistical analysis for the difference of each group was conducted, and then Student T-test was performed for the statistical analysis. High Fat Diet control (HFD) was compared with Normal diet control (ND), but High Fat Diet + silymarin treatment group (HFD+Sily) or High Fat Diet + red ginseng concentrate treatment group (HFD+Gx), having enhanced Compound K, was compared with High Fat Diet control (HFD). The result is shown in Table 3 below.

[Table 3]

|  | ND | HFD | HFD+Sily | HFD+Gx100 | HFD+Gx250 | HFD+Gx500 |
|---|---|---|---|---|---|---|
| The weight of the liver (g) | 0.78± 0.07 | 0.88± 0.05* | 0.87± 0.08 | 0.83± 0.08 | 0.81± 0.04 | 0.92± 0.05 |
| The liver/weight (w/w %) | 2.9± 0.4 | 2.6± 0.1 | 2.8± 0.2 | 2.5± 0.1 | 2.6± 0.2 | 2.8± 0.2 |
| Normal diet vs. High Fatty diet: *($p<0.05$), **($p<0.01$)<br>High Fatty diet vs. High Fatty diet + sample: #($p<0.05$), ##($p<0.01$) | | | | | | |

In order to consider a difference in the weight of the liver due to the weight of body, the weight of the liver was divided by the weight of body of each of experimental animals and was converted it to liver/body % (w/w %), and again the average value was calculated for each of the groups.

As a result of determining the weight of the liver removed when sacrificing the animals and comparing it with each other, the average liver weight for High Fatty diet control (HFD) was 0.88±0.05g and exhibited a significant increasing of about 13% over the average weight of liver (0.78± 0.07g) for Normal diet control (ND). It can be considered that the fat was accumulated in the liver by taking High Fatty diet and thus obesity phenomenon of the liver was appeared.

## 1-2. Histopathological change

Fatty liver was induced by treating High Fatty diet (HFD) for 11 weeks. When observing with the naked eyes, a color of the liver for High Fatty diet (HFD) control was exhibited a yellowish color relative to that of Normal diet (ND) control. It can be determined that a considerable accumulation of fat in the liver tissue was occurred by the High Fatty diet.

After completing the experiment of 11 weeks, the histopathological change for the liver tissue was identified.

In order to identify the histopathological change for the liver tissue, the sample of the liver removed when sacrificing the animal was fixed on a slide, Oil Red O staining for identifying the lipid in the cell and Hematoxylin and Eosin staining (H&E staining) for histochemically analyzing the fat tissue were performed and then identified them through a microscope. The results were represented in Figs. 2a and 2b.

As seen from FIG. 2a, when identifying it through the microscope after the Oil Red O staining, lipid drops stained as red color were discovered in everywhere of cells of High Fatty diet control (HFD).

In FIG. 2b, lots of great circular vesicles meaning the accumulation of lipid in hepatocytes were appeared in the liver of High Fatty Diet control (HFD), and in the case of the red ginseng concentrate having enhanced Compound K experiment group (HFD + Gx), the size of the lipid drop was greatly reduced over that of the lipid drop in the High Fatty Diet control (HFD). In addition, it could be identified that the accumulation of neutral lipid was reduced over the High Fatty Diet control (HFD).

## 1-3. Change of the biochemical index in the blood

When a part of the liver function is damaged by the fatty liver, the change in the relevant biochemical indices appears. This experiment selected the major liver functional indices which can be used in relation to the diagnosis of the fatty liver and analyzed the difference in each of groups.

As noticed from FIG. 3, in the case of the contents of TG, the neutral lipid in the blood, High Fatty Diet (HFD) control exhibited the significantly high value of about 2.1 times over Normal Diet (ND) control, and in the case of red ginseng concentrate having enhanced Compound K experiment group (HFD + Gx), it is exhibited that the contents of the neutral lipid was significantly reduced over High Fatty Diet (HFD) control regardless of the concentration of the red ginseng concentrate having enhanced Compound K.

This result is interpreted that the red ginseng concentrate having enhanced Compound K can provide the direct affect to the lipid metabolism in the blood.

## 1-4. Change of the content of neutral lipid in the liver tissue

The content of the neutral lipid (Triglycerides, TG) in the liver tissue was analyzed, and the results are exhibited in FIG. 4.

As in FIG. 4, the average TG content of High Fatty Diet control (HFD) was increased 2 times or more over that of Normal Diet control (ND), and the red ginseng concentrate having enhanced Compound K experimental groups (HFD+Gx100, HFD+Gx250, HFD+Gx500) exhibited the tendency that the content of TG was reduced again.

Such result means that the red ginseng concentrate having enhanced Compound K has the function regulating the neutral lipid metabolism in the blood and the liver tissue.

## Experiment example 2: Analysis of gene expression for the proteins related to the lipid metabolism in the liver tissue

As the first step for studying the mechanism regulating that the red ginseng concentrate having enhanced Compound K regulates the lipid metabolism in the liver tissue, the gene expression for the major proteins involving lipogenesis and lipolysis in the liver tissue was analyzed by Real-time RT-PCR method, and the results were exhibited in FIG. 5.

SREBP-1c (Sterol Regulatory Element Binding transcription factor 1), FAS (Fatty Acid Synthase), ACC (Acety-CoA Carboxylase), LXR$\alpha$ (Liver X Receptor Alpha), as the proteins regulating de novo Lipogenesis in the hepatopocytes, were analyzed.

PCR of 36B4 (acidic ribosomal phosphoprotein PO), Housekeeping gene was used as an Internal standard of the analysis for the gene expression.

SREBP-1c is a transcription factor regulating the expression of lipid biosynthesis genes such as FAS, ACC, etc. in the hepatocytes, and is also the major target gene for LXR$\alpha$ actuating as Lipid sensor in the hepatocytes. Therefore, when LXR$\alpha$ is activated by any external signal, the expression of lipid biosynthesis enzyme genes such as FAS, ACC, etc., is activated through a promotion of SREBP-1 c expression.

As indicated in FIG. 5, as a result of Real-time RT-PCR experiment, the expression of Lipogenic genes in the hepatocytes was significantly activated by HFD, and it can be noticed that the expression was also inhibited by the treatment of the red ginseng concentrate having enhanced Compound K. It can be deduced from said results that red ginseng concentrate having enhanced Compound K inhibits LXR$\alpha$-mediated SREBBP-1c induction mechanism, and thus, has the function controlling the biosynthesis of lipid activated by HFD.

**Claims**

1. A pharmaceutical composition for treating and preventing non-alcoholic fatty liver disease, which comprises red ginseng concentrate having enhanced Compound K.

2. The pharmaceutical composition according to claim 1, wherein the red ginseng concentrate having enhanced Compound K are comprised in an amount of 0.1 to 99 % by weight to the total weight of the pharmaceutical composition.

3. The pharmaceutical composition according to claim 1, wherein the content of the Compound K is enhanced to the level of 0.5 to 1.5mg/g.

4. A health functional food comprising red ginseng concentrate having enhanced Compound K as an effective ingredient.

5. The health functional food according to claim 4, wherein the red ginseng concentrate having enhanced Compound K is comprised in an amount of 0.1 to 99 % by weight to the total of weight of the health functional food.

6. The health functional food according to claim 1, wherein the content of the Compound K is enhanced to the level of 0.5 to 1.5mg/g.

【FIG. 1】

```
┌─────────────────────────────────────┐
│            Red ginseng              │
└─────────────────────────────────────┘
              │
              │   Extraction by water or thyl alchohol
              │   (70℃ ~ 80℃, 15hr, repeat 3 times)
┌─────────────────────────────────────┐
│            Concentration            │
└─────────────────────────────────────┘
              │
┌─────────────────────────────────────┐
│    Enzyme reaction(Cytolase PCL5)   │
└─────────────────────────────────────┘
              │   (56℃, 24~85hr)
┌─────────────────────────────────────┐
│        Inactivation of enzyme       │
└─────────────────────────────────────┘
              │
┌─────────────────────────────────────┐
│            Concentration            │
└─────────────────────────────────────┘
```

【FIG. 2a】

【FIG. 2b】

【FIG. 3】

【FIG. 4】

【FIG. 5】

Europäisches
Patentamt

European
Patent Office

Office européen
des brevets

## EUROPEAN SEARCH REPORT

Application Number

EP 15 19 4312

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/328951 A1 (SHIM HYEON JAE [KR] ET AL) 6 November 2014 (2014-11-06) <br> * paragraphs [0100] - [0104], [0126], [0130], [0140] * | 1-6 | INV. <br> A61K36/258 <br> A61P1/16 <br> A61K31/575 <br> A23L33/105 |
| X | LEE HAE-UNG ET AL: "Hepatoprotective effect of ginsenoside Rb1 and compound K on tert-butyl hydroperoxide-induced liver injury.", <br> LIVER INTERNATIONAL : OFFICIAL JOURNAL OF THE INTERNATIONAL ASSOCIATION FOR THE STUDY OF THE LIVER OCT 2005, <br> vol. 25, no. 5, October 2005 (2005-10), pages 1069-1073, XP002755327, <br> ISSN: 1478-3223 <br> * the whole document * | 1-6 | |
| X | KIM MOON-SUN ET AL: "Compound K modulates fatty acid-induced lipid droplet formation and expression of proteins involved in lipid metabolism in hepatocytes", <br> LIVER INTERNATIONAL, <br> vol. 33, no. 10, November 2013 (2013-11), pages 1583-1593, XP002755328, <br> * the whole document * | 1-6 | TECHNICAL FIELDS SEARCHED (IPC) <br><br> A61K <br> A23L |
| X | LEE H U ET AL: "Hepatoprotective effect of 20(S)-ginsenosides Rg3 and its metabolite 20(S)-ginsenoside Rh2 on tert-butyl hydroperoxide-induced liver injury", <br> MEDICINAL & AROMATIC PLANTS ABSTRACTS, SCIENTIFIC PUBLISHERS, SCIENTIFIC PUBLISHERS, NEW DELHI - INDIA, <br> vol. 28, no. 1, <br> 1 February 2006 (2006-02-01), XP018010982, <br> ISSN: 0250-4367 <br> * the whole document * | 1-6 | |

-/--

The present search report has been drawn up for all claims

| Place of search | Date of completion of the search | Examiner |
|---|---|---|
| Munich | 10 March 2016 | Greif, Gabriela |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

Europäisches
Patentamt

European
Patent Office

Office européen
des brevets

## EUROPEAN SEARCH REPORT

Application Number

EP 15 19 4312

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DO YEON KIM ET AL: "Compound K, Intestinal Metabolite of Ginsenoside, Attenuates Hepatic Lipid Accumulation via AMPK Activation in Human Hepatoma Cells", JOURNAL OF AGRICULTURAL AND FOOD CHEMISTRY, vol. 57, no. 4, 25 February 2009 (2009-02-25), pages 1532-1537, XP055255061, US ISSN: 0021-8561, DOI: 10.1021/jf802867b * the whole document * | 1-6 | |
| X,P | IGAMI KENTARO ET AL: "Hepatoprotective effect of fermented ginseng and its major constituent compound K in a rat model of paracetamol (acetaminophen)-induced liver injury.", THE JOURNAL OF PHARMACY AND PHARMACOLOGY APR 2015, vol. 67, no. 4, April 2015 (2015-04), pages 565-572, XP002755329, ISSN: 2042-7158 * the whole document * | 1-6 | |
| | | | **TECHNICAL FIELDS SEARCHED (IPC)** |

The present search report has been drawn up for all claims

| Place of search | Date of completion of the search | Examiner |
|---|---|---|
| Munich | 10 March 2016 | Greif, Gabriela |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 15 19 4312

This annex lists the patent family members relating to the patent documents cited in the above-mentioned European search report.
The members are as contained in the European Patent Office EDP file on
The European Patent Office is in no way liable for these particulars which are merely given for the purpose of information.

10-03-2016

| Patent document cited in search report | Publication date | Patent family member(s) | Publication date |
|---|---|---|---|
| US 2014328951 A1 | 06-11-2014 | KR 20130038168 A<br>US 2014328951 A1 | 17-04-2013<br>06-11-2014 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020130085970

**Non-patent literature cited in the description**

- **HASEGAWA, H. et al.** *Planta Medica,* 1996, vol. 62, 453-457
- **HASEGAWA, H. et al.** *Planta Medica,* 1997, vol. 63 (5), 436-440
- **KITAGAWA et al.** *Yakugaku Zasshi.,* 1983, vol. 103, 612-622
- **KWON et al.** *J.Chromatography A.,* 2001, vol. 921, 335-339
- **PARK.** *Food Ind. Nutr.,* 2004, vol. 9, 23-27
- **HAN et al.** *Planta medica,* 1982, vol. 44, 146-149
- **BAE et al.** *Arch Pharm Res,* 2004, vol. 27 (1), 61-67
- **CHEN et al.** *Chem. Pharm. Bull.,* 1987, vol. 35, 1653-1655
- **IM et al.** *Kor J Ginseng Sci.,* 1995, vol. 19, 291-294
- **ANUFRIEV et al.** *Carbohydr Res.,* 1997, vol. 304, 179-182
- Korean College of Pharmacy Conference. Explanation of the Korean Pharmacopoeia. Munsung Company, 1989, vol. 5, 33, , 48